# EUROPEAN PATENT SPECIFICATION

(11) **EP 3 344 188 B1**
(45) Date of publication and mention of the grant of the patent: **24.09.2025**
(21) Application number: 16763672.9
(22) Date of filing: 31.08.2016
(51) Int. Cl.: A61F 2/24

(54) **TRANSCATHETER VALVE PROSTHESES HAVING A SEALING COMPONENT FORMED FROM TISSUE HAVING AN ALTERED EXTRACELLULAR MATRIX**
TRANSKATHETER-HERZKLAPPENPROTHESEN MIT EINER DICHTUNGSKOMPONENTE, DIE AUS GEWEBE MIT EINER VERÄNDERTEN EXTRAZELLULÄREN MATRIX GEFORMT IST
PROTHÈSES DE VALVULES TRANSCATHÉTER À COMPOSANT D'ÉTANCHÉITÉ FORMÉ À PARTIR DE TISSUS COMPRENANT UNE MATRICE EXTRACELLULAIRE MODIFIÉE

(30) Priority: 02.09.2015 US 201514843128
(43) Date of publication of application: 11.07.2018
(73) Proprietor: Medtronic Vascular Inc., Santa Rosa, CA 95403 (US)
(72) Inventor: WANG, Wei, Santa Rosa, CA 95403 (US); MCKINLEY, Laura, Santa Rosa, CA 95403 (US); TIEN, Tracey, Santa Rosa, CA 95403 (US); WONG, Benjamin, Santa Rosa, CA 95403 (US); OLNEY, Karl, Santa Rosa, CA 95403 (US)
(74) Representative: Zimmermann & Partner Patentanwälte mbB
(86) International application number: PCT/US2016/049634
(87) International publication number: WO 2017/040614

(56) References cited:
- EP-A1- 2 893 905
- WO-A1-2014/140230
- US-A1- 2013 331 929
- US-B2- 9 144 663

## Description

### FIELD OF THE INVENTION

The present invention relates in general to transcatheter valve prostheses, and more particularly to a transcatheter valve prosthesis having one or more components for preventing paravalvular leakage.

### BACKGROUND OF THE INVENTION

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Recently, flexible prosthetic valves supported by stent structures that can be delivered percutaneously using a catheter-based delivery system have been developed for heart and venous valve replacement. These prosthetic valves may include either self-expanding or balloon-expandable stent structures with valve leaflets attached to the interior of the stent structure. The prosthetic valve can be reduced in diameter, by crimping onto a balloon catheter or by being contained within a sheath component of a delivery catheter, and advanced through the venous or arterial vasculature. Once the prosthetic valve is positioned at the treatment site, for instance within an incompetent native valve, the stent structure may be expanded to hold the prosthetic valve firmly in place. One example of a stented prosthetic valve is disclosed in U.S. Pat. No. 5,957,949 to Leonhardt et al. entitled "Percutaneous Placement Valve Stent". Another example of a stented prosthetic valve for a percutaneous pulmonary valve replacement procedure is described in U.S. Patent Application Publication No. 2003/0199971 A1 and U.S. Patent Application Publication No. 2003/0199963 A1, both filed by Tower et al..

Although transcatheter delivery methods have provided safer and less invasive methods for replacing a defective native heart valve, leakage between the implanted prosthetic valve and the surrounding native tissue is a recurring problem. Leakage sometimes occurs due to the fact that minimally invasive and percutaneous replacement of cardiac valves typically does not involve actual physical removal of the diseased or injured heart valve. Rather, the replacement stented prosthetic valve is delivered in a compressed condition to the valve site, where it is expanded to its operational state within the diseased valve. Calcified or diseased native leaflets are pressed to the side walls of the native valve by the radial force of the stent frame of the prosthetic valve. These calcified leaflets do not allow complete conformance of the stent frame with the native valve and can be a source of paravalvular leakage (PVL). Significant pressure gradients across the valve cause blood to leak through the gaps between the implanted prosthetic valve and the calcified anatomy.

Embodiments hereof are related to transcatheter valve prostheses having one or more components attached thereto or integrated thereon to address and prevent paravalvular leakage. EP 2 893 905 A1 relates to a heart valve support ring.

### BRIEF SUMMARY OF THE INVENTION

The invention is as defined in claim 1.

Embodiments hereof relate to a transcatheter valve prosthesis including a stent having a compressed state for delivery within a vasculature and an expanded state for deployment within a native heart valve, a prosthetic valve component disposed within and secured to the stent, and a sealing component coupled to the stent. The sealing component is formed from a tissue having an altered extracellular matrix that includes at least one weakened connection such that the tissue is configured to swell upon contact with a fluid.

According to another embodiment hereof, a transcatheter valve prosthesis includes a stent having a compressed state for delivery within a vasculature and an expanded state for deployment within a native heart valve, a prosthetic valve component disposed within and secured to the stent, and a sealing component coupled to the stent. The sealing component is formed from a tissue having an altered extracellular matrix. The tissue having the altered extracellular matrix has an expanded state upon contact with a fluid and a compressed state for delivery within a vasculature. A thickness of the tissue having the altered extracellular matrix in the expanded state is at least 50% greater than a thickness of the tissue having a non-altered extracellular matrix in an unloaded state in which no force is applied thereto and a thickness of the tissue having the altered extracellular matrix in the compressed state is at least 25% less than the thickness of the tissue having the non-altered extracellular matrix in the unloaded state.

According to another embodiment hereof, a transcatheter valve prosthesis includes a stent having a compressed state for delivery within a vasculature and an expanded state for deployment within a native heart valve, a prosthetic valve component disposed within and secured to the stent, and a sealing component coupled to the stent. The sealing component is formed from pericardial tissue and the pericardial tissue has an expanded state upon contact with a fluid and a compressed state for delivery within a vasculature, wherein the pericardial tissue has an altered extracellular matrix that includes at least one weakened connection and the pericardial tissue having the altered extracellular matrix transforms from the compressed state to the expanded state *in situ* when blood infiltrates the at least one weakened connection.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIG. 1 is a side view illustration of an exemplary transcatheter valve prosthesis for use in embodiments hereof.
FIG. 1A is a top view illustration of the transcatheter valve prosthesis of FIG. 1.
FIG. 1B is a side view illustration of an alternative configuration of a transcatheter valve prosthesis for use in embodiments hereof.
FIG. 1C is a side view illustration of an alternative configuration of a transcatheter valve prosthesis for use in embodiments hereof.
FIG. 2 is a side view illustration of the transcatheter valve prosthesis of FIG. 1 implanted within a native valve annulus.
FIG. 3 is a side view illustration of a transcatheter valve prosthesis including a sealing component around an outer surface thereof, wherein the sealing component is formed from a tissue having an altered extracellular matrix that includes at least one weakened connection such that the tissue is configured to swell upon contact with a fluid, the sealing component being shown in an expanded state.
FIG. 4 is a perspective sectional view of layers of heart tissue.
FIG. 5 is a schematic side sectional view of a portion of a pericardial tissue having a non-altered extracellular matrix, the pericardial tissue being shown in an unloaded state in which no force is applied thereto.
FIG. 5A is an enlarged view of a portion of FIG. 5.
FIG. 5B is a schematic enlarged side sectional view of a portion of pericardial tissue having an altered extracellular matrix.
FIG. 6 is a side view illustration of the transcatheter valve prosthesis of FIG. 3, wherein the sealing component is shown in a compressed state within a delivery sheath for delivery within a vasculature.
FIG. 6A is a cross-sectional view illustration of the transcatheter valve prosthesis of FIG. 6 taken along line A-A of FIG. 6, wherein the delivery sheath is not shown for sake of clarity only.
FIG. 7 is a side view illustration of the transcatheter valve prosthesis of FIG. 3, wherein the sealing component is shown *in situ* in the expanded state upon contact with a fluid.
FIG. 7A is a cross-sectional view illustration of the transcatheter valve prosthesis of FIG. 7 taken along line A-A of FIG. 7.
FIG. 8 is a side view illustration of a transcatheter valve prosthesis including a sealing component around an outer surface thereof according to another embodiment hereof, the sealing component including a skirt that forms an open-ended pocket, wherein the sealing component is formed from a tissue having an altered extracellular matrix that includes at least one weakened connection such that the tissue is configured to swell upon contact with a fluid and the sealing component is shown in the expanded state.
FIG. 9 is a side view illustration of a transcatheter valve prosthesis including a sealing component around an outer surface thereof according to another embodiment hereof, the sealing component including a skirt that forms a compartment and a filter positioned over an opening formed on the skirt, wherein the sealing component is formed from a tissue having an altered extracellular matrix that includes at least one weakened connection such that the tissue is configured to swell upon contact with a fluid and the sealing component is shown in the expanded state.
FIG. 10 is a side view illustration of a transcatheter valve prosthesis including a sealing component around an outer surface thereof according to another embodiment hereof, the sealing component including a plurality of compartments that extend around the entire perimeter of the transcatheter valve prosthesis, wherein the sealing component is formed from a tissue having an altered extracellular matrix that includes at least one weakened connection such that the tissue is configured to swell upon contact with a fluid and the sealing component is shown in the expanded state.
FIG. 11 is a side view illustration of a transcatheter valve prosthesis including a sealing component attached thereto according to another embodiment hereof, the sealing component including a plurality of compartments that extend around a portion of the perimeter of the transcatheter valve prosthesis, wherein the sealing component is formed from a tissue having an altered extracellular matrix that includes at least one weakened connection such that the tissue is configured to swell upon contact with a fluid and the sealing component is shown in the expanded state.

### DETAILED DESCRIPTION OF THE INVENTION

Specific embodiments of the present invention are now described with reference to the figures, wherein like reference numbers indicate identical or functionally similar elements. If utilized herein, the terms "distal" or "distally" refer to a position or in a direction away from the heart and the terms "proximal" and "proximally" refer to a position near or in a direction toward the heart. The following detailed description is merely exemplary in nature and is not intended to limit the invention or the application and uses of the invention. Although the description of the invention is in the context of treatment of heart valves, the invention may also be used where it is deemed useful in other valved intraluminal sites that are not in the heart. For example, the present invention may be applied to venous valves as well. Furthermore, there is no intention to be bound by any expressed or implied theory presented in the preceding technical field, background, brief summary or the following detailed description.

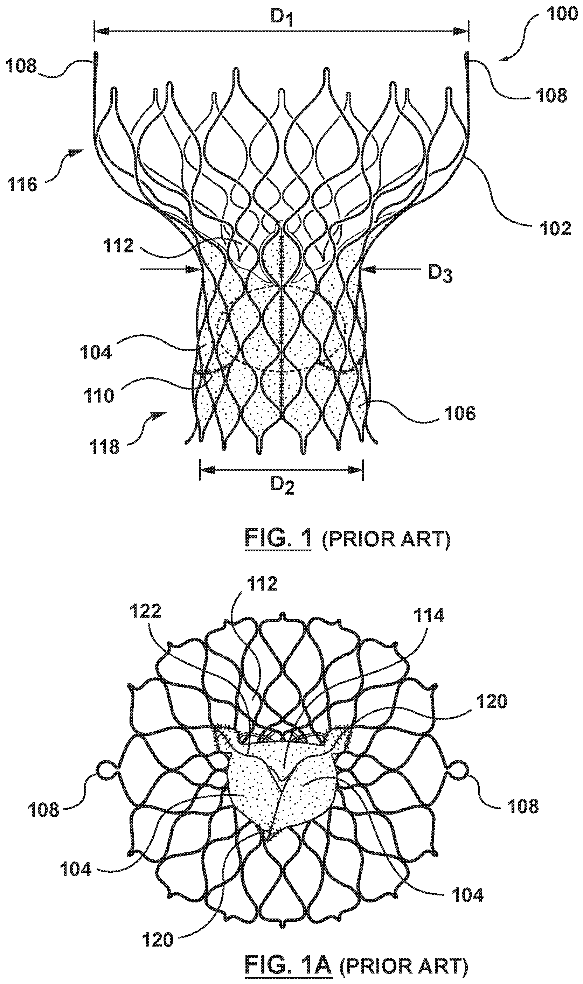

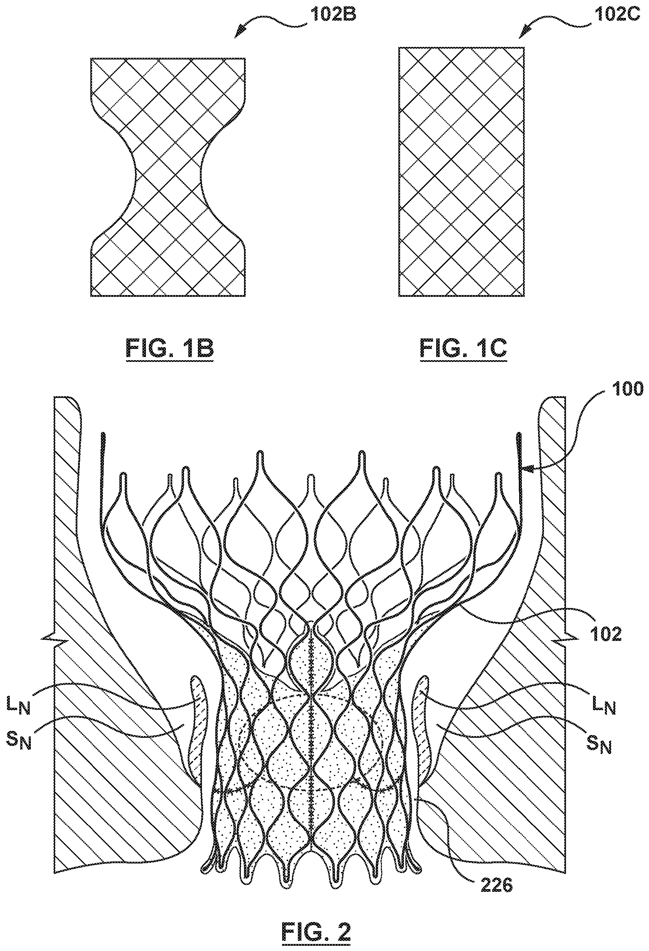

FIG. 1 depicts an exemplary transcatheter valve prosthesis 100 shown in its expanded or deployed configuration. Transcatheter valve prosthesis 100 is illustrated herein in order to facilitate description of the methods and devices to prevent and/or repair paravalvular leakage according to embodiments hereof. It is understood that any number of alternate heart valve prostheses can be used with the methods and devices described herein. Transcatheter valve prosthesis 100 is merely exemplary and is described in more detail in U.S. Patent Application Pub. No. 2011/0172765 to Nguyen et al..

Transcatheter valve prosthesis 100 includes an expandable stent or frame 102 that supports a prosthetic valve component within the interior of stent 102. Stent 102 is a generally tubular support structure or scaffold that defines a lumen there-through. In this embodiment, stent 102 is a unitary tubular component having a plurality of side openings 112, which may be formed by a laser-cut manufacturing method from a cylindrical tube and/or another conventional stent forming method as would be understood by one of ordinary skill in the art. In an embodiment, side openings 112 may be diamond shaped or of another shape. It will be understood by one of ordinary skill in the art that the illustrated configurations of stent 102 are exemplary and stent 102 may have alternative patterns or configurations. For example, in another embodiment (not shown), stent 102 may include a series of independent or separate sinusoidal patterned rings coupled to each other to form a tubular component. In embodiments hereof, stent 102 is self-expanding to return to an expanded deployed state from a compressed or constricted delivery state and may be made from stainless steel, a pseudo-elastic metal such as a nickel titanium alloy or Nitinol, or a so-called super alloy, which may have a base metal of nickel, cobalt, chromium, or other metal. "Self-expanding" as used herein means that a structure/component has a mechanical memory to return to the expanded or deployed configuration. Mechanical memory may be imparted to the wire or tubular structure that forms stent 102 by thermal treatment to achieve a spring temper in stainless steel, for example, or to set a shape memory in a susceptible metal alloy, such as nitinol, or a polymer, such as any of the polymers disclosed in U.S. Pat. Appl. Pub. No. 2004/0111111 to Lin. Alternatively, transcatheter valve prosthesis 100 may be balloon-expandable as would be understood by one of ordinary skill in the art.

In the embodiment depicted in FIGS. 1 and 1A, stent 102 of valve prosthesis 100 has a deployed configuration including an enlarged or flared first end or section 116 and a second end or section 118. Enlarged first section 116 has nominal deployed diameter D₁ and second section 118 has nominal deployed diameter D₂. Each section of stent 102 may be designed with a number of different configurations and sizes to meet the different requirements of the location in which it may be implanted. When configured as a replacement for an aortic valve, second section 118 functions as an inflow end of transcatheter valve prosthesis 100 and extends into and anchors within the aortic annulus of a patient's left ventricle, while first section 116 functions as an outflow end of transcatheter valve prosthesis 100 and is positioned in the patient's ascending aorta. When configured as a replacement for a mitral valve, enlarged first section 116 functions as an inflow end of transcatheter valve prosthesis 100 and is positioned in the patient's left atrium, while second section 118 functions as an outflow end of transcatheter valve prosthesis 100 and extends into and anchors within the mitral annulus of a patient's left ventricle. For example, U.S. Patent Application Publication Nos. 2012/0101572 to Kovalsky et al*.* and 2012/0035722 to Tuval illustrate heart valve prostheses configured for placement in a mitral valve. Each section of stent 102 may have the same or different cross-section which may be for example circular, ellipsoidal, rectangular, hexagonal, rectangular, square, or other polygonal shape, although at present it is believed that circular or ellipsoidal may be preferable when the valve prosthesis is being provided for replacement of the aortic or mitral valve. As alternatives to the deployed configuration of FIGS. 1 and 1A, the stent/valve support frame may have an hourglass configuration or profile 102B shown in FIG. 1B, a generally tubular configuration or profile 102C as shown in FIG. 1C, or other stent configuration or shape known in the art for valve replacement. Stent 102 also may include eyelets 108 that extend from first end 116 thereof for use in loading the transcatheter valve prosthesis 100 into a delivery catheter (not shown).

As previously mentioned, transcatheter valve prosthesis 100 includes a prosthetic valve component within the interior of stent 102. The prosthetic valve component is capable of blocking flow in one direction to regulate flow there through via valve leaflets 104 that may form a bicuspid or tricuspid replacement valve. FIG. 1A is an end view of FIG. 1 and illustrates an exemplary tricuspid valve having three leaflets 104, although a bicuspid leaflet configuration may alternatively be used in embodiments hereof. More particularly, if transcatheter valve prosthesis 100 is configured for placement within a native valve having three leaflets such as the aortic, tricuspid, or pulmonary valves, transcatheter valve prosthesis 100 includes three valve leaflets 104. If transcatheter valve prosthesis 100 is configured for placement within a native valve having two leaflets such as the mitral valve, transcatheter valve prosthesis 100 includes two valve leaflets 104. Valve leaflets 104 are sutured or otherwise securely and sealingly attached to the interior surface of stent 102 and/or graft material 106 which encloses or lines a portion of stent 102 as would be known to one of ordinary skill in the art of prosthetic tissue valve construction. Referring to FIG. 1, leaflets 104 are attached along their bases 110 to graft material 106, for example, using sutures or a suitable biocompatible adhesive. Adjoining pairs of leaflets are attached to one another at their lateral ends to form commissures 120, with free edges 122 of the leaflets forming coaptation edges that meet in area of coaptation 114.

Leaflets 104 may be made of pericardial material; however, the leaflets may instead be made of another material. Natural tissue for replacement valve leaflets may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as leaflets 104 include DACRON^{®} polyester commercially available from Invista North America S.A.R.L. of Wilmington, DE, other cloth materials, nylon blends, polymeric materials, and vacuum deposition nitinol fabricated materials. One polymeric material from which the leaflets can be made is an ultra-high molecular weight polyethylene material commercially available under the trade designation DYNEEMA from Royal DSM of the Netherlands. With certain leaflet materials, it may be desirable to coat one or both sides of the leaflet with a material that will prevent or minimize overgrowth. It is further desirable that the leaflet material is durable and not subject to stretching, deforming, or fatigue.

Graft material 106 may also be a natural or biological material such as pericardium or another membranous tissue such as intestinal submucosa. Alternatively, graft material 106 may be a low-porosity woven fabric, such as polyester, Dacron fabric, or PTFE, which is attached or coupled to an interior or exterior surface of the stent. In an embodiment, graft material 106 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side. These and other appropriate cardiovascular fabrics are commercially available from Bard Peripheral Vascular, Inc. of Tempe, Ariz., for example. In an embodiment shown in FIG. 1, graft material 106 is coupled to and covers an inner circumferential surface of stent 102 and extends from leaflets bases 110 to second end 118 of transcatheter valve prosthesis.

Delivery of transcatheter valve prosthesis 100 may be accomplished via a percutaneous transfemoral approach or a transapical approach directly through the apex of the heart via a thoracotomy, or may be positioned within the desired area of the heart via different delivery methods known in the art for accessing heart valves. During delivery, if self-expanding, the prosthetic valve remains compressed until it reaches a target diseased native heart valve, at which time the transcatheter valve prosthesis 100 can be released from the delivery catheter and expanded *in situ* via self-expansion. The delivery catheter is then removed and transcatheter valve prosthesis 100 remains deployed within the native target heart valve. Alternatively, transcatheter valve prosthesis 100 may be balloon-expandable and delivery thereof may be accomplished via a balloon catheter as would be understood by one of ordinary skill in the art. Transcatheter valve prosthesis 100 may be self-expandable, balloon-expandable, mechanically-expandable, or some combination thereof.

FIG. 2 is a side view illustration of transcatheter valve prosthesis 100 implanted within a native aortic heart valve, which is shown in section, having native leaflets L_{N} and corresponding native sinuses S_{N}. When transcatheter valve prosthesis 100 is deployed within the valve annulus of a native heart valve, stent 102 expands within native valve leaflets L_{N} of the patient's defective valve, retaining the native valve leaflets in a permanently open state. The native valve annulus may include surface irregularities on the inner surface thereof, and as a result one or more gaps or cavities/crevices 226 may be present or may form between the perimeter of transcatheter valve prosthesis 100 and the native valve annulus. For example, calcium deposits may be present on the native valve leaflets (e.g., stenotic valve leaflets) and/or shape differences may be present between the native heart valve annulus and prosthesis 100. More particularly, in some cases native annuli are not perfectly rounded and have indentations corresponding to the commissural points of the native valve leaflets. As a result, a prosthesis having an approximately circular cross-section does not provide an exact fit in a native valve. These surface irregularities, whatever their underlying cause, can make it difficult for conventional prosthetic valves to form a blood tight seal between the prosthetic valve and the inner surface of the valve annulus, causing undesirable paravalvular leakage and/or regurgitation at the implantation site.

Embodiments hereof relate to a transcatheter valve prosthesis having a sealing component that functions to occlude or fill gaps between the perimeter of the transcatheter valve prosthesis and the native valve annulus, thereby reducing, minimizing, or eliminating leaks there-between. The sealing component is formed from a tissue having an altered extracellular matrix. More particularly, the sealing component is formed from a tissue having an altered extracellular matrix that includes at least one weakened connection such that the tissue is configured to swell upon contact with a fluid. The tissue having an altered extracellular matrix transforms from a compressed state for delivery within a vasculature to an expanded state *in situ* when blood infiltrates or flows within the at least one weakened connection.

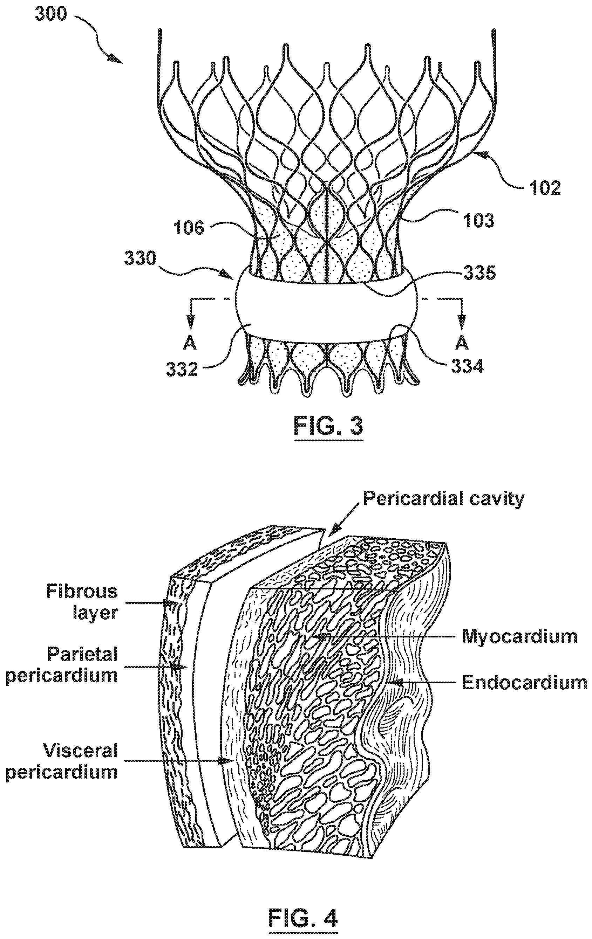

More particularly, with reference to FIG. 3, a transcatheter valve prosthesis 300 according to an embodiment hereof is shown. In FIG. 3, sealing component 330 is shown in an expanded state. Similar to transcatheter valve prosthesis 100, transcatheter valve prosthesis 300 includes tubular stent 102, graft material 106 coupled to and covering an inner circumferential surface of stent 102, and a prosthetic valve component that includes leaflets 104 disposed within and secured to stent 102. However, unlike transcatheter valve prosthesis 100, transcatheter valve prosthesis 300 also includes sealing component 330 which is a skirt 332 coupled to and covering an outer circumferential surface of stent 102 for sealing and preventing paravalvular leakage. Skirt 332 has a first end or edge 334 and an opposing second end or edge 335 that are both attached to outer surface 103 of stent 102. First and second opposing edges 334, 335 of skirt 332 may be attached to stent 102 by any suitable means known to those skilled in the art, for example and not by way of limitation, welding, adhesive, suture/stitches, or mechanical coupling. Sealing component 330 is formed from a tissue having an altered extracellular matrix that includes at least one weakened connection such that the tissue is configured to swell upon contact with a fluid.

More particularly, in an embodiment hereof, skirt 332 is formed from pericardial tissue, such as but not limited to bovine or equine pericardial tissue, having an altered extracellular matrix. With reference to FIG. 4, which is a perspective sectional view of heart tissue, pericardial tissue includes a visceral pericardium and a parietal pericardium which are separated by a pericardial cavity. The visceral pericardium is of very thin translucent tissue most adjacent the heart and surrounds the heart and the roots of the great blood vessels. The parietal pericardium is a thicker membrane of multi-layered connective tissue covered with adipose or fat tissue which is removed (i.e., peeled off) when harvested. The remaining multi-layered connective tissue of the parietal pericardium (i.e., the parietal pericardium without the adipose or fat tissue) includes a plurality of compact or dense layers of collagen having interspersed elastic fibers. Collagen is a protein which is the major fibrous component of skin, bone, tendon, cartilage, ligaments and blood vessels. Collagen is rich in glycine and also contains proline, hydroxyproline, lysine and hydroxylysine. The remaining multi-layered connective tissue of the parietal pericardium (i.e., the parietal pericardium without the adipose or fat tissue) is utilized as a basis or source for the tissue of skirt 332.

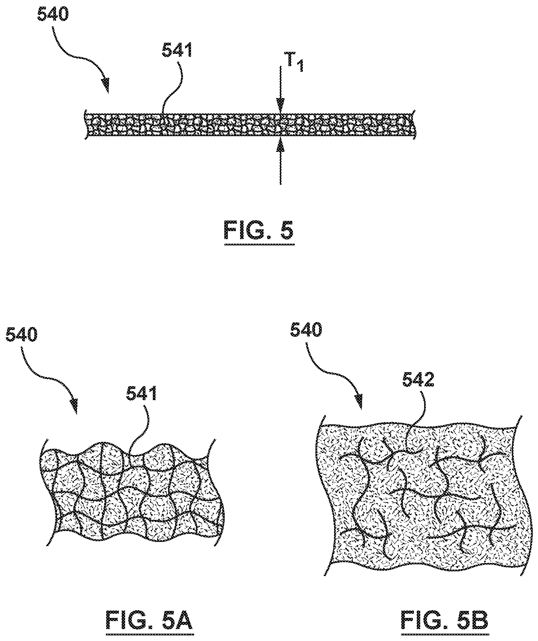

FIG. 5 is a side sectional view of a portion of a tissue 540 having a non-altered extracellular matrix 541. As shown in FIG. 5, tissue 540 having non-altered extracellular matrix 541 has a thickness T₁ in the unloaded state in which no force is applied thereto. Stated another way, T₁ is the thickness of tissue 540 before disruption or alteration of the extracellular matrix and with no compressive forces applied thereto. In an embodiment hereof, thickness T₁ ranges from 0.08 mm to 1.0 mm depending on the tissue source and factors such as species, age, and the like.

FIG. 5A is an enlarged side sectional view of a portion of tissue 540 having non-altered extracellular matrix 541, while FIG. 5B is an enlarged side sectional view of a portion of tissue 540 having an altered extracellular matrix 542. As described above, tissue 540 is derived from connective tissue of parietal pericardium that primarily contains fibrous collagen. Altered extracellular matrix 542 of tissue 540 includes at least one weakened connection such that the tissue is configured to swell upon contact with a fluid. As used herein, weakened connection refers to a loosened, broken, separated, weakened, disrupted, or otherwise reorganized fiber, bond, link, or other structure within the connective tissue of parietal pericardium. Stated another way, one or more of the fibers or other structure of the connective tissue of parietal pericardium is purposely or deliberately loosened, broken, separated, weakened, disrupted, or otherwise reorganized in order to form altered extracellular matrix 542 having at least one weakened connection. Thus, embodiments hereof relate to modifying the connections or strength of one or more of the fibers or other structure within the connective tissue of parietal pericardium but do not alter or change the material makeup of the parietal pericardium. Although not required, in an embodiment hereof, the weakened connection may create or result in gaps, breaks, fractures, cracks, cavities, openings, and/or layer separations within altered extracellular matrix 542 of tissue 540. The weakened connection of altered extracellular matrix 542 causes tissue 540 to swell upon contact with a fluid when the fluid infiltrates or flows within the weakened connection. Accordingly, when positioned *in situ,* tissue 540 having altered extracellular matrix 542 expands or swells and thus has an increased thickness. More particularly, chemical and/or mechanical means alter the tissue's extracellular matrix so that tissue 540 having altered extracellular matrix 542 expands upon contact with a fluid and then remains in this expanded state to permanently set the increased thickness. Further, as will be explained in more detail herein, altered extracellular matrix 542 configures tissue 540 to expand upon contact with fluid environment but does not reduce compressibility of the tissue such that the delivery profile of transcatheter valve prosthesis 300 is not adversely affected. When expanded *in situ* due to the increased thickness of tissue 540 having altered extracellular matrix 542, sealing component 330 is configured to adapt to the irregular geometry of a native valve annulus to attain better apposition of transcatheter valve prosthesis 300 without changing the packing density of transcatheter valve prosthesis 300 inside of a delivery catheter or sheath.

Altered extracellular matrix 542 of tissue 540 may be formed via one or more tissue processing methods. In an embodiment hereof, altered extracellular matrix 542 of tissue 540 is mechanically altered by mechanical shearing to form the at least one weakened connection. For example, one side of tissue 540 is held stationary while the opposing side of tissue 540 is moved or rubbed laterally in order to fracture, break or otherwise weaken one or more fibers within the connective tissue of parietal pericardium and thereby form the at least one weakened connection by mechanical shearing. In another embodiment hereof, altered extracellular matrix 542 of tissue 540 is mechanically altered by osmotic pressure to form the at least one weakened connection. For example, tissue 540 is subjected or immersed into hypotonic solution such that resultant pressure on the surface thereof would disrupt or reorganize one or more fibers within the connective tissue of parietal pericardium and thereby form the at least one weakened connection by osmotic pressure. More particularly, when osmotic pressure is utilized to form the at least one weakened connection, the hypotonic solution causes one or more fibers within the connective tissue of parietal pericardium to expand or inflate by, for example, causing one or more originally loose fibers or connections or become tighter or taut and thereby resulting in expansion or inflation of the tissue. In another embodiment hereof, altered extracellular matrix 542 of tissue 540 is mechanically altered by freezing to form the at least one weakened connection. For example, opposing sides of tissue 540 are subjected to freezing temperatures at different rates in order to cause internal matrix damage in which one or more fibers within the connective tissue of parietal pericardium fracture, break or otherwise weaken and thereby form the at least one weakened connection by freezing. In another embodiment hereof, altered extracellular matrix 542 of tissue 540 is chemically altered by chemical cleaving to form the at least one weakened connection. For example, tissue 540 is subjected to chemical reactions that fracture, break or otherwise weaken one or more fibers within the connective tissue of parietal pericardium and thereby form the at least one weakened connection by chemical cleaving. In another embodiment hereof, altered extracellular matrix 542 of tissue 540 is partially digested using enzymes such as but not limited to collagenase or elastase that fracture, break or otherwise weaken one or more fibers within the connective tissue of the parietal pericardium and thereby form the at least one weakened connection by enzymatic digestion. As previously stated, altered extracellular matrix 542 of tissue 540 may be formed via one of the above-described tissue processing methods, or altered extracellular matrix 542 of tissue 540 may be formed via a combination of one or more of the above-described tissue processing methods such as but not limited to a combination of mechanical shearing and osmotic pressure.

Notably, after altered extracellular matrix 542 of tissue 540 is formed via one or more of the above-described tissue processing methods, tissue 540 having altered extracellular matrix 542 is then expanded via submersing it into an formaldehyde or other preservative hypotonic solution for storage and preservation thereof. Thus, tissue 540 having altered extra-cellular matrix 542 is stored or fixed in its expanded configuration. Prior to being submersed into the preservative solution for storage thereof (or stated another way, when in an unloaded state in which no force is applied thereof), tissue 540 having altered extracellular matrix 542 may be between 0 and 20% thicker than thickness T₁ of tissue 540 having non-altered extracellular matrix 541 as shown and described with respect to FIG. 5A, depending upon which type(s) of tissue processing method(s) are utilized for forming altered extracellular matrix 542. In an embodiment, tissue 540 having altered extracellular matrix 542 may be stored in the preservative solution as a planar or flat component in its expanded configuration, removed from the preservative solution for compression thereof as a planar component, and then assembled onto transcatheter valve prosthesis 300 in its compressed configuration to form sealing component 330 when it is desired to prepare transcatheter valve prosthesis 300 for delivery. In another embodiment, tissue 540 having altered extracellular matrix 542 may be assembled onto transcatheter valve prosthesis 300 to form sealing component 330 prior to submersion into the preservative solution for storage thereof and transcatheter valve prosthesis 300 having sealing component 330 thereon may be compressed for delivery when desired.

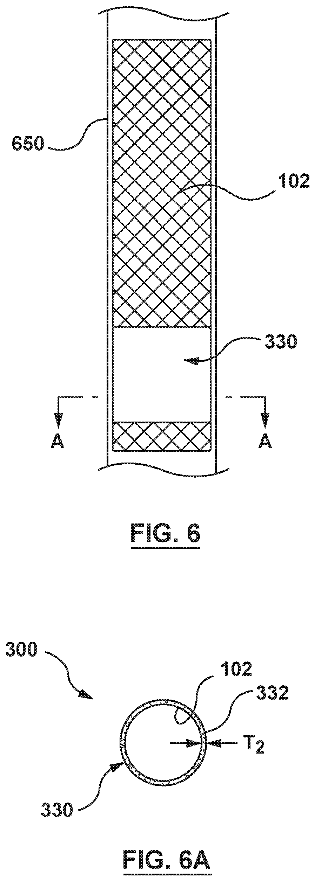

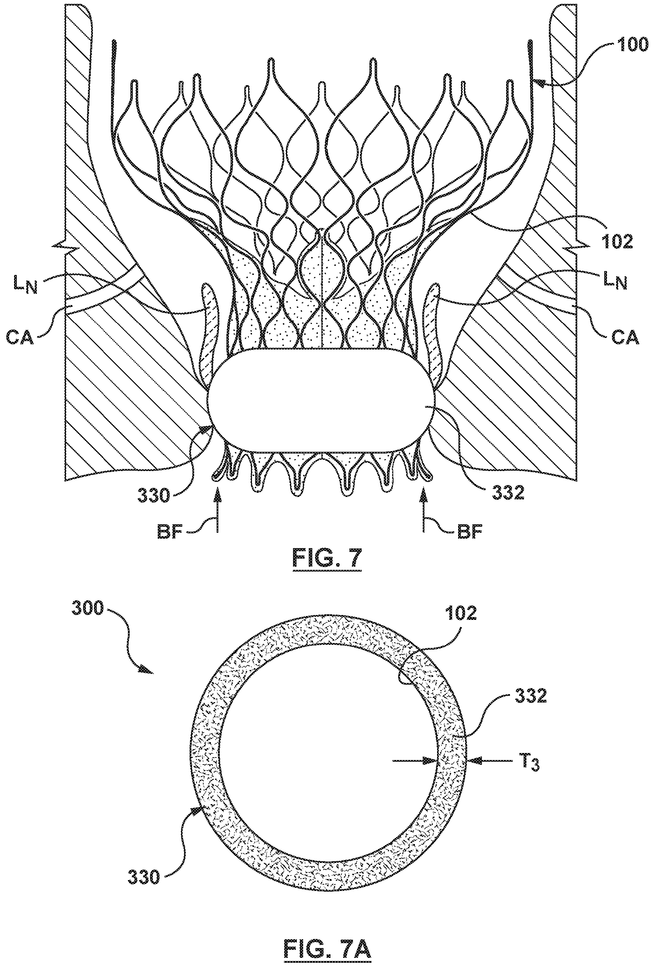

Sealing component 330 has a compressed state for delivery within a vasculature as shown in FIG. 6 and FIG. 6A, and the expanded state *in situ* upon contact with a fluid as shown in FIG. 7 and FIG. 7A. Tissue 540 having altered extracellular matrix 542 of sealing component 330 is configured to transform from the compressed state to the expanded state *in situ* when blood infiltrates or flows into the at least one weakened connection. More particularly, as shown in FIG. 6 and FIG. 6A, transcatheter valve prosthesis 300 is compressed within a delivery sheath or catheter 650 (not shown in the cross-sectional view of FIG. 6A for illustrative purposes only) during delivery within a vasculature and skirt 332 (formed from tissue 540 having altered extracellular matrix 542) has a thickness T₂ in the compressed state. In an embodiment hereof, tissue 540 having altered extracellular matrix 542 has at least the same compressibility as tissue 540 having non-altered extracellular matrix 541. Stated another way, the altered extracellular matrix of tissue 540 does not reduce compressibility of the tissue. Tissue 540 having altered extracellular matrix 542 thus is configured such that the materials' ability to compress down to a relatively thin state without an increase in compression resistance (i.e., no or low reaction force upon compression) is preserved. As such, transcatheter valve prosthesis 300 may be crimped or compressed down to a size that may be loaded into delivery sheath or catheter 650. In an embodiment, delivery sheath or catheter 650 may have a delivery size or profile of 18F. In an embodiment hereof, thickness T₂ ranges from 0.005 mm to 0.20 mm. Thickness T₂ of tissue 540 having altered extracellular matrix 542 in the compressed state is at least 25% less than thickness T₁ of tissue 540 having non-altered extracellular matrix 541 in the unloaded state in which no force is applied thereto. In an embodiment hereof, thickness T₂ of tissue 540 having altered extracellular matrix 542 in the compressed state is between 40% and 60% less than thickness T₁ of tissue 540 having non-altered extracellular matrix 541 in the unloaded state in which no force is applied thereto.

Further, in another embodiment hereof, tissue 540 having altered extracellular matrix 542 has improved compressibility relative to tissue 540 having non-altered extracellular matrix 541. Stated another way, the altered extracellular matrix of tissue 540 increases the compressibility of the tissue. Thickness T₂ of tissue 540 having altered extracellular matrix 542 in the compressed state may be up to 50% more compressible than tissue 540 having non-altered extracellular matrix 541. Stated another way, in an embodiment hereof, less force (i.e., up to 50% less force) is required to compress tissue 540 having altered extracellular matrix 542 compared to the force required to equally compress tissue 540 having non-altered extracellular matrix 541. For example, when altered extracellular matrix 542 of tissue 540 is mechanically altered by mechanical shearing to form the at least one weakened connection, tissue 540 having altered extracellular matrix 542 has improved compressibility.

In FIG. 7 and FIG. 7A, sealing component 330 is shown in the expanded state *in situ* upon contact with a fluid. As shown in the cross-sectional view of FIG. 7A, skirt 332 has a thickness T₃ in the expanded state *in situ* upon contact with a fluid. Stated another way, T₃ is the thickness of tissue 540 with altered extracellular matrix 542 in the expanded state after recovering from compression and in contact with a fluid. In an embodiment hereof, thickness T₃ ranges from 0.20 mm to 2.0 mm. In an embodiment, thickness T₃ of tissue 540 having altered extracellular matrix 542 in the expanded state that is at least 50% greater than thickness T₁ in the unloaded state in which no force is applied thereto. Further, in another embodiment, thickness T₃ of tissue 540 having altered extracellular matrix 542 in the expanded state that is at least 75% greater than thickness T₁ of tissue 540 having non-altered extracellular matrix 541 in the unloaded state in which no force is applied thereto and may be over 100% greater than thickness T₁ of tissue 540 having non-altered extracellular matrix 541 in the unloaded state in which no force is applied thereto. When in the expanded state, skirt 332 functions to block any retrograde flow within the native valve, thereby preventing undesired regurgitation and preventing blood stagnation in and around the native valve sinuses. In addition, when transcatheter valve prosthesis 300 is deployed, skirt 332 in the expanded state fills any/all gaps or cavities/crevices between the outer surface of stent 102 and native valve tissue such that blood flow through the target gap or cavity is occluded or blocked, or stated another way blood is not permitted to flow there-through. Skirt 332 functions as a continuous circumferential seal around transcatheter valve prosthesis 300 to block or prevent blood flow around the outer perimeter of the prosthesis, thereby minimizing and/or eliminating any paravalvular leakage at the implantation site.

In the embodiment of FIG. 3, sealing component 330 is coupled to outer surface 103 of transcatheter valve prosthesis 300 adjacent to inflow or distal end 318 thereof. When deployed, sealing component 330 may be positioned *in situ* at the native valve annulus, slightly above the valve annulus, slightly below the valve annulus, or some combination thereof. Since the sealing component is coupled to outer surface 103 of transcatheter valve prosthesis 300, longitudinal placement and/or the size and shape thereof is flexible and may be adjusted or adapted according to each application and to a patient's unique needs. For example, depending on the anatomy of the particular patient, the sealing component may be positioned on transcatheter valve prosthesis 300 so that *in situ* the sealing component is positioned between transcatheter valve prosthesis 300 and the interior surfaces of the native valve leaflets, between transcatheter valve prosthesis 300 and the interior surfaces of the native valve annulus, and/or between transcatheter valve prosthesis 300 and the interior surfaces of the left ventricular outflow track (LVOT). Further, it will be understood by one of ordinary skill in the art that the length of sealing component 330 may vary according to application and skirt 332 may extend over a longer or shorter portion of transcatheter valve prosthesis 300.

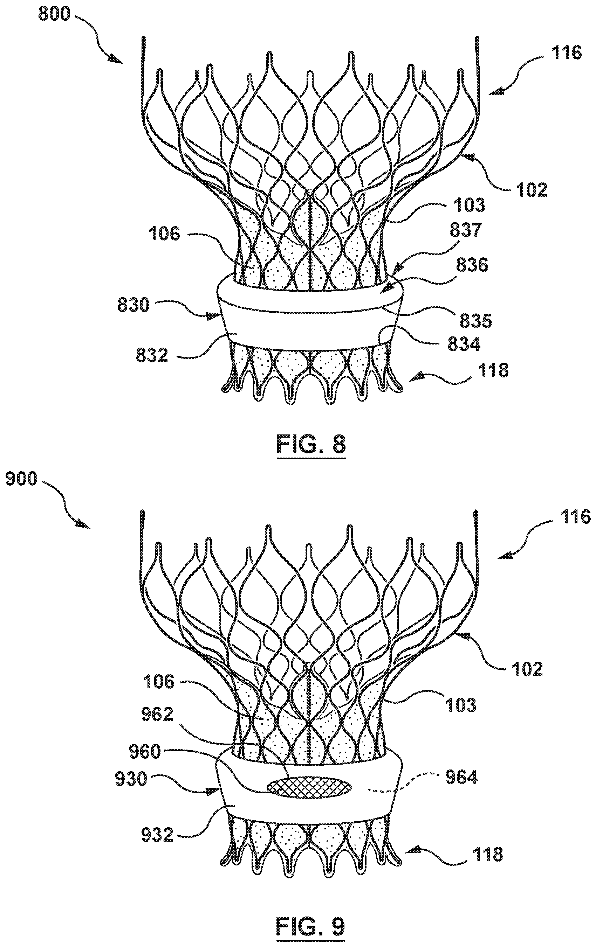

Sealing components according to embodiments hereof may have various configurations. For example, FIG. 8 illustrates a transcatheter valve prosthesis 800 having a sealing component 830 that includes a skirt 832 which forms an annular open-ended pocket 836. Similar to sealing component 330, sealing component 830 is formed from a tissue having an altered extracellular matrix that includes at least one weakened connection such that the tissue is configured to swell upon contact with a fluid. Sealing component 830 is shown in FIG. 8 in an expanded state. Skirt 832 is a flap of material having a first end or edge 834 attached to outer surface 103 of stent 102 and an opposing second end or edge 835 not coupled to stent 102 to form pocket 836 having open end 837 between second edge 835 of skirt 832 and outer surface 103 of stent 102. Stated another way, second edge 835 of skirt 832 is radially spaced apart from outer surface 103 of stent 102 and annular pocket 836 is formed between skirt 832 and stent 102, which includes graft material 106 that encloses or lines a portion of stent 102. First edge 834 of skirt 832 may be attached to stent 102 by any suitable means known to those skilled in the art, for example and not by way of limitation, welding, adhesive, suture, or mechanical coupling. *In situ,* blood flow between the perimeter of transcatheter valve prosthesis 800 and the native valve annulus blood is permitted to flow into pocket 836 to thereby fill pocket 836 with blood. For example, retrograde blood flow may flow into pocket 836 when transcatheter valve prosthesis 800 is configured for placement within an aorta valve. As pocket 836 fills with blood, skirt 832 (which forms the outer surface of pocket 836) radially or outwardly expands into and substantially fills any/all gaps or cavities/crevices between outer surface 103 of stent 102 and native valve tissue. Stated another way, once pocket 836 is filled with blood, sealing component 830 functions as a continuous circumferential seal around transcatheter valve prosthesis 800 to block or prevent blood flow around the outer perimeter of the prosthesis, thereby minimizing and/or eliminating any paravalvular leakage at the implantation site. Although FIG. 8 illustrates open-ended pocket 836 of sealing component 830 oriented to catch retrograde blood flow, it would be obvious to one of ordinary skill in the art that pocket 836 may be inverted to catch antegrade flow rather than retrograde flow.

In another embodiment hereof, sealing component 830 may include an expandable control ring (not shown) coupled to the second or unattached edge of skirt 832 which operates to radially extend or deploy unattached second edge 835 of skirt 832 outwardly away from stent 102 as described in U.S. Patent Application Publication No. 2014/0194981 to Menk et al., Application No. 13/738,376 (Attorney Docket No. P0041527.USU2). The expandable control ring may be formed from a self-expanding material or may have an adjustable diameter that may be varied *in situ* to selectively extend the unattached second edge 835 of skirt 832 outwardly away from the outer surface of the transcatheter valve prosthesis.

In another example, FIG. 9 illustrates a transcatheter valve prosthesis 900 having a sealing component 930 that includes a skirt 932. Similar to sealing component 330, sealing component 930 is formed from a tissue having an altered extracellular matrix that includes at least one weakened connection such that the tissue is configured to swell upon contact with a fluid. Sealing component 930 is shown in FIG. 9 in an expanded state. Skirt 932 is similar to skirt 332 except that skirt 932 includes a filter 960 positioned over an opening 962 formed on the skirt as described in U.S. Patent Application No. 14/731,629 to Keogh (Attorney Docket No. C00008766.S0). The filtered opening is configured to permit blood flow there-through and to trap emboli in the blood flow within a compartment 964 formed by skirt 932. Stated another way, blood is permitted to flow into compartment 964 via the filtered opening but any emboli or blood clots that may form or develop within the compartment are trapped therein and therefore prevented from being released into a patient's bloodstream.

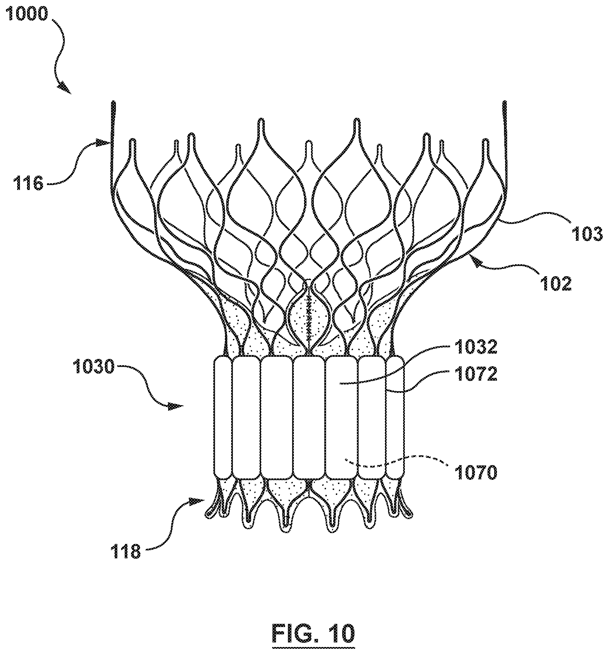

In another example, FIG. 10 illustrates a transcatheter valve prosthesis 1000 having a sealing component 1030 that includes a skirt 1032. Similar to sealing component 330, sealing component 1030 is formed from a tissue having an altered extracellular matrix that includes at least one weakened connection such that the tissue is configured to swell upon contact with a fluid. Sealing component 1030 is shown in FIG. 10 in an expanded state. Skirt 1032 is similar to skirt 332 except that skirt 1032 includes a plurality of compartments 1070 positioned around the exterior of the transcatheter valve prosthesis. A plurality of dividers or seams 1072 may be provided on skirt 1032 to form the plurality of compartments 1070 positioned around stent 102. The compartments can be formed in any number, size, and/or shape around stent 102. Although sealing component 1030 is shown in FIG. 10 as being positioned to extend around the full perimeter or outer surface 103 of stent 102, sealing component 1030 may include a plurality of spaced-apart compartments that do not touch or abut against each other. For example, in an embodiment (not shown), a plurality of spaced-apart compartments are attached to an outer surface of stent 102 and configured to be disposed within gaps formed at the commissural points of native valve leaflets. Further, although sealing component 1030 is described as being formed with a skirt 1032 having seams 1072, it will be understood by one of ordinary skill in the art that the plurality of compartments may be formed via a plurality of distinct or individual skirts rather than a single integral skirt having seams or dividers to form the plurality of compartments.

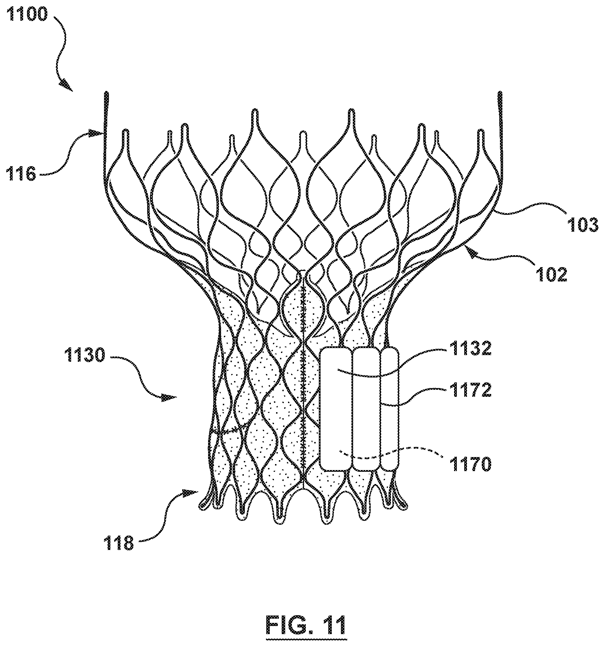

Although the above embodiments illustrate an annular sealing component, the sealing component is not required to extend around the entire perimeter of a transcatheter valve prosthesis. For example, FIG. 11 illustrates a transcatheter valve prosthesis 1100 having a sealing component 1130 that includes a skirt 1132. Similar to sealing component 330, sealing component 1130 is formed from a tissue having an altered extracellular matrix that includes at least one weakened connection such that the tissue is configured to swell upon contact with a fluid. Sealing component 1130 is shown in FIG. 11 in an expanded state. Similar to sealing component 1030, sealing component 1130 includes a plurality of pockets or compartments 1170 formed by a plurality of seams or dividers 1172. In this embodiment, however, the plurality of compartments 1170 are positioned around a portion of the perimeter of stent 102 and do not extend around the entire perimeter of the stent.

Although embodiments depicted herein illustrate sealing components integrated onto a transcatheter valve prosthesis configured for implantation within an aortic valve, it would be obvious to one of ordinary skill in the art that the sealing components as described herein may be integrated onto a transcatheter valve prosthesis configured for implantation implanted within other heart valves, such as a mitral valve, tricuspid valve, or a pulmonary valve. The transcatheter valve prosthesis may be designed with a number of different configurations and sizes to meet the different requirements of the location in which it may be implanted.

Further, although embodiments depicted herein illustrate sealing components integrated onto an outer or exterior circumferential surface of a transcatheter valve prosthesis, sealing components formed from a tissue having an altered extracellular matrix that includes at least one weakened connection such that the tissue is configured to swell upon contact with a fluid may alternatively and/or additionally be integrated onto an inner or interior circumferential surface of the transcatheter valve prosthesis. For example, in another embodiment hereof, graft material coupled to a stent or scaffold of an implantable prosthesis such as graft material 106 described above may be formed from a tissue having an altered extracellular matrix that includes at least one weakened connection such that the tissue is configured to swell upon contact with a fluid.

While various embodiments according to the present invention have been described above, it should be understood that they have been presented by way of illustration and example only, and not limitation. It will be apparent to persons skilled in the relevant art that various changes in form and detail can be made therein without departing from the scope of the invention. Thus, the breadth and scope of the present invention should not be limited by any of the above-described exemplary embodiments, but should be defined only in accordance with the appended claims It will also be understood that each feature of each embodiment discussed herein, and of each reference cited herein, can be used in combination with the features of any other embodiment.

## Claims

1. A transcatheter valve prosthesis (300, 800, 900, 1000, 1100) comprising:
a stent (102) having a compressed state for delivery within a vasculature and an expanded state for deployment within a native heart valve;
a prosthetic valve component disposed within and secured to the stent (102); and
a sealing component (330, 830, 930, 1030, 1130) coupled to the stent (102), wherein the sealing component (330, 830, 930, 1030, 1130) is formed from a tissue having an altered extracellular matrix (542) that includes at least one weakened connection such that the tissue is configured to swell upon contact with a fluid; and wherein the altered extracellular matrix (542) has been mechanically altered to form the at least one weakened connection by a process selected from the group consisting of mechanical shearing, osmotic pressure, and freezing.

2. The transcatheter valve prosthesis (300, 800, 900, 1000, 1100) of claim 1, wherein the tissue having the altered extracellular matrix (542) has an expanded state upon contact with a fluid and a compressed state for delivery within a vasculature.

3. The transcatheter valve prosthesis (300, 800, 900, 1000, 1100) of claim 2, wherein the tissue having the altered extracellular matrix (542) has a thickness in the expanded state that is at least 50% greater than a thickness of the tissue having a non-altered extracellular matrix (541) in an unloaded state when no force is applied thereto.

4. The transcatheter valve prosthesis (300, 800, 900, 1000, 1100) of claim 2, wherein the tissue having the altered extracellular matrix (542) is configured to transform from the compressed state to the expanded state *in situ* when blood infiltrates the at least one weakened connection.

5. The transcatheter valve prosthesis (300, 800, 900, 1000, 1100) of claim 2, wherein the tissue having the altered extracellular matrix (542) has at least the same compressibility as the tissue having a non-altered extracellular matrix (541).

6. The transcatheter valve prosthesis (300, 800, 900, 1000, 1100) of claim 1, wherein the sealing component (330, 830, 930, 1030, 1130) is a skirt (332, 832, 932, 1032, 1132) that encircles an exterior of the stent (102).

7. The transcatheter valve prosthesis (300, 800, 900, 1000, 1100) of claim 1, wherein the stent (102) includes a tubular scaffold and the sealing component (330, 830, 930, 1030, 1130) is coupled to the scaffold.

8. The transcatheter valve prosthesis (300, 800, 900, 1000, 1100) of claim 1, wherein the tissue having the altered extracellular matrix (542) is pericardial tissue.

9. The transcatheter valve prosthesis (300, 800, 900, 1000, 1100) of claim 2,wherein a thickness of the tissue having the altered extracellular matrix (542) in the expanded state is at least 50% greater than a thickness of the tissue having a non-altered extracellular matrix (541) in an unloaded state when no force is applied thereto and a thickness of the tissue having the altered extracellular matrix (542) in the compressed state is at least 25% less than the thickness of the tissue having the non-altered extracellular matrix (541) in the unloaded state.

10. The transcatheter valve prosthesis (300, 800, 900, 1000, 1100) of claim 9, wherein the altered extracellular matrix (542) includes at least one weakened connection and the tissue having the altered extracellular matrix (542) is configured to transform from the compressed state to the expanded state *in situ* when blood infiltrates the at least one weakened connection.

11. The transcatheter valve prosthesis (300, 800, 900, 1000, 1100) of claim 9, wherein the thickness of the tissue having the altered extracellular matrix (542) in the expanded state is at least 75% greater than the thickness of the tissue having the non-altered extracellular matrix (541) in the unloaded state.

12. The transcatheter valve prosthesis (300, 800, 900, 1000, 1100) of claim 9, wherein the sealing component (330, 830, 930, 1030, 1130) is a skirt (332, 832, 932, 1032, 1132) that encircles an exterior of the stent (102).

13. The transcatheter valve prosthesis (300, 800, 900, 1000, 1100) of claim 9, wherein the stent (102) includes a tubular scaffold and the sealing component (330, 830, 930, 1030, 1130) is coupled to the scaffold.

## Patentansprüche

1. Transkatheter-Klappenprothese (300, 800, 900, 1000, 1100), umfassend:
einen Stent (102), der einen komprimierten Zustand für eine Einführung innerhalb eines Gefäßsystems und einen expandierten Zustand für eine Entfaltung innerhalb einer nativen Herzklappe aufweist;
eine Klappenprothesekomponente, die innerhalb des Stents (102) angeordnet und an diesem fixiert ist; und
eine Dichtungskomponente (330, 830, 930, 1030, 1130), die mit dem Stent (102) gekoppelt ist, wobei die Dichtungskomponente (330, 830, 930, 1030, 1130) aus einem Gewebe, das eine veränderte extrazelluläre Matrix (542) aufweist, gebildet ist, die mindestens eine geschwächte Verbindung derart einschließt, dass das Gewebe konfiguriert ist, um bei Kontakt mit einer Flüssigkeit aufzuquellen; und wobei die veränderte extrazelluläre Matrix (542) mechanisch verändert wurde, um die mindestens eine geschwächte Verbindung durch einen Prozess zu bilden, der aus der Gruppe ausgewählt ist, die aus mechanischem Scheren, osmotischem Druck und Einfrieren besteht.

2. Transkatheter-Klappenprothese (300, 800, 900, 1000, 1100) nach Anspruch 1, wobei das Gewebe, das die veränderte extrazelluläre Matrix (542) aufweist, bei Kontakt mit einer Flüssigkeit einen expandierten Zustand und bei der Einführung innerhalb eines Gefäßes einen komprimierten Zustand aufweist.

3. Transkatheter-Klappenprothese (300, 800, 900, 1000, 1100) nach Anspruch 2, wobei das Gewebe, das die veränderte extrazelluläre Matrix (542) aufweist, in dem expandierten Zustand eine Dicke aufweist, die mindestens 50 % größer ist als eine Dicke des Gewebes, das eine unveränderte extrazelluläre Matrix (541) aufweist, in einem unbelasteten Zustand, wenn keine Kraft darauf ausgeübt wird.

4. Transkatheter-Klappenprothese (300, 800, 900, 1000, 1100) nach Anspruch 2, wobei das Gewebe, das die veränderte extrazelluläre Matrix (542) aufweist, konfiguriert ist, um von dem komprimierten Zustand in den expandierten Zustand *in situ* überzugehen wenn Blut in die mindestens eine geschwächte Verbindung eindringt.

5. Transkatheter-Klappenprothese (300, 800, 900, 1000, 1100) nach Anspruch 2, wobei das Gewebe, das die veränderte extrazelluläre Matrix (542) aufweist, mindestens die gleiche Kompressibilität aufweist wie das Gewebe, das eine unveränderte extrazellulären Matrix (541) aufweist.

6. Transkatheter-Klappenprothese (300, 800, 900, 1000, 1100) nach Anspruch 1, wobei die Dichtungskomponente (330, 830, 930, 1030, 1130) eine Schürze (332, 832, 932, 1032, 1132) ist, die eine Außenseite des Stents (102) umgibt.

7. Transkatheter-Klappenprothese (300, 800, 900, 1000, 1100) nach Anspruch 1, wobei der Stent (102) ein röhrenförmiges Gerüst einschließt und die Dichtungskomponente (330, 830, 930, 1030, 1130) mit dem Gerüst gekoppelt ist.

8. Transkatheter-Klappenprothese (300, 800, 900, 1000, 1100) nach Anspruch 1, wobei das Gewebe, das die veränderte extrazelluläre Matrix (542) aufweist, Perikardgewebe ist.

9. Transkatheter-Klappenprothese (300, 800, 900, 1000, 1100) nach Anspruch 2, wobei eine Dicke des Gewebes, das die veränderte extrazelluläre Matrix (542) aufweist, in dem expandierten Zustand mindestens 50 % größer ist als eine Dicke des Gewebes, das eine unveränderte extrazelluläre Matrix (541) aufweist, in einem unbelasteten Zustand, wenn keine Kraft darauf ausgeübt wird, und eine Dicke des Gewebes, das die veränderte extrazelluläre Matrix (542) aufweist, in dem komprimierten Zustand mindestens 25 % geringer ist als die Dicke des Gewebes, das die unveränderte extrazellulären Matrix (541) aufweist, in dem unbelasteten Zustand.

10. Transkatheter-Klappenprothese (300, 800, 900, 1000, 1100) nach Anspruch 9, wobei die veränderte extrazelluläre Matrix (542) mindestens eine geschwächte Verbindung einschließt und das Gewebe, das die veränderte extrazelluläre Matrix (542) aufweist, konfiguriert ist, um von dem komprimierten Zustand in den expandierten Zustand *in situ* überzugehen wenn Blut in die mindestens eine geschwächte Verbindung eindringt.

11. Transkatheter-Klappenprothese (300, 800, 900, 1000, 1100) nach Anspruch 9, wobei die Dicke des Gewebes, das die veränderte extrazelluläre Matrix (542) aufweist, in dem expandierten Zustand mindestens 75 % größer ist als die Dicke des Gewebes, das die unveränderte extrazelluläre Matrix (541) aufweist, in dem unbelasteten Zustand.

12. Transkatheter-Klappenprothese (300, 800, 900, 1000, 1100) nach Anspruch 9, wobei die Dichtungskomponente (330, 830, 930, 1030, 1130) eine Schürze (332, 832, 932, 1032, 1132) ist, die eine Außenseite des Stents (102) umgibt.

13. Transkatheter-Klappenprothese (300, 800, 900, 1000, 1100) nach Anspruch 9, wobei der Stent (102) ein röhrenförmiges Gerüst einschließt und die Dichtungskomponente (330, 830, 930, 1030, 1130) mit dem Gerüst gekoppelt ist.

## Revendications

1. Prothèse valvulaire transcathéter (300, 800, 900, 1000, 1100) comprenant :
une endoprothèse (102) ayant un état comprimé destiné à l'administration à l'intérieur d'un système vasculaire et un état expansé pour un déploiement à l'intérieur d'une valvule cardiaque native ;
un composant de valvule prothétique disposé à l'intérieur de l'endoprothèse (102) et fixé à celle-ci ; et
un composant d'étanchéité (330, 830, 930, 1030, 1130) accouplé à l'endoprothèse (102), dans lequel le composant d'étanchéité (330, 830, 930, 1030, 1130) est formé à partir d'un tissu ayant une matrice extracellulaire altérée (542) qui comporte au moins une connexion affaiblie de telle sorte que le tissu est conçu pour gonfler au contact d'un fluide ; et dans lequel la matrice extracellulaire altérée (542) a été modifiée mécaniquement pour former l'au moins une connexion affaiblie par un procédé choisi parmi le groupe constitué de cisaillement mécanique, de pression osmotique et de congélation.

2. Prothèse valvulaire transcathéter (300, 800, 900, 1000, 1100) selon la revendication 1, dans laquelle le tissu ayant la matrice extracellulaire altérée (542) a un état expansé au contact d'un fluide et un état comprimé pour l'administration à l'intérieur d'un système vasculaire.

3. Prothèse valvulaire transcathéter (300, 800, 900, 1000, 1100) selon la revendication 2, dans laquelle le tissu ayant la matrice extracellulaire altérée (542) a une épaisseur à l'état expansé qui est au moins 50 % plus grande qu'une épaisseur du tissu ayant une matrice extracellulaire non altérée (541) à l'état non chargé lorsqu'aucune force n'est appliquée à celui-ci.

4. Prothèse valvulaire transcathéter (300, 800, 900, 1000, 1100) selon la revendication 2, dans laquelle le tissu ayant la matrice extracellulaire altérée (542) est conçu pour passer de l'état comprimé à l'état expansé *in situ* lorsque le sang s'infiltre dans l'au moins une connexion affaiblie.

5. Prothèse valvulaire transcathéter (300, 800, 900, 1000, 1100) selon la revendication 2, dans laquelle le tissu ayant la matrice extracellulaire altérée (542) a au moins la même compressibilité que le tissu ayant une matrice extracellulaire non altérée (541).

6. Prothèse valvulaire transcathéter (300, 800, 900, 1000, 1100) selon la revendication 1, dans laquelle le composant d'étanchéité (330, 830, 930, 1030, 1130) est une jupe (332, 832, 932, 1032, 1132) qui entoure un extérieur de l'endoprothèse (102).

7. Prothèse valvulaire transcathéter (300, 800, 900, 1000, 1100) selon la revendication 1, dans laquelle l'endoprothèse (102) comporte un échafaudage tubulaire et le composant d'étanchéité (330, 830, 930, 1030, 1130) est accouplé à l'échafaudage.

8. Prothèse valvulaire transcathéter (300, 800, 900, 1000, 1100) selon la revendication 1, dans laquelle le tissu ayant la matrice extracellulaire altérée (542) est un tissu péricardique.

9. Prothèse valvulaire transcathéter (300, 800, 900, 1000, 1100) selon la revendication 2, dans laquelle une épaisseur du tissu ayant la matrice extracellulaire altérée (542) à l'état expansé est supérieure d'au moins 50 % à une épaisseur du tissu ayant une matrice extracellulaire non altérée (541) à l'état déchargé lorsqu'aucune force ne lui est appliquée et une épaisseur du tissu ayant la matrice extracellulaire altérée (542) à l'état comprimé est inférieure d'au moins 25 % à l'épaisseur du tissu ayant la matrice extracellulaire non altérée (541) à l'état déchargé.

10. Prothèse valvulaire transcathéter (300, 800, 900, 1000, 1100) selon la revendication 9, dans laquelle la matrice extracellulaire altérée (542) comporte au moins une connexion affaiblie et le tissu ayant la matrice extracellulaire altérée (542) est conçu pour passer de l'état comprimé à l'état expansé *in situ* lorsque le sang s'infiltre dans l'au moins une connexion affaiblie.

11. Prothèse valvulaire transcathéter (300, 800, 900, 1000, 1100) selon la revendication 9, dans laquelle l'épaisseur du tissu ayant la matrice extracellulaire altérée (542) à l'état expansé est supérieure d'au moins 75 % à l'épaisseur du tissu ayant la matrice extracellulaire non altérée (541) à l'état déchargé.

12. Prothèse valvulaire transcathéter (300, 800, 900, 1000, 1100) selon la revendication 9, dans laquelle le composant d'étanchéité (330, 830, 930, 1030, 1130) est une jupe (332, 832, 932, 1032, 1132) qui entoure un extérieur de l'endoprothèse (102).

13. Prothèse valvulaire transcathéter (300, 800, 900, 1000, 1100) selon la revendication 9, dans laquelle l'endoprothèse (102) comporte un échafaudage tubulaire et le composant d'étanchéité (330, 830, 930, 1030, 1130) est accouplé à l'échafaudage.
